# EUROPEAN PATENT APPLICATION

(11) **EP 2 584 041 A1**
(43) Date of publication of application: **24.04.2013**
(21) Application number: 12189201.2
(22) Date of filing: 19.10.2012
(51) Int. Cl.: C12N 15/113, A61K 31/713, A61P 3/10, C12N 9/24

(54) **Composition for preventing or treating diabetes comprising nad glycohydrolase inhibitor as active ingredient**

(30) Priority: 21.10.2011 KR 20110108216
(71) Applicant: Industrial Cooperation Foundation Chonbuk National University, Jeonju -Si, Jeollabuk-Do 561-191 (KR)
(72) Inventor: Kim, Uh-Hyun, Jeonju-si Jeonbuk 561-794 (KR); Nam, Tae-Sik, Suwon-si Gyeonggi-do 443-380 (KR); Park, Kwang-Hyun, Jeonju-si Jeonbuk 561-794 (KR); Kim, Byung-Ju, Jeonju-si Jeonbuk 561-784 (KR)
(74) Representative: Buchet, Anne

(57) **Abstract**

The present invention provides a composition for preventing or treating diabetes, comprising an NAD glycohydrolase (NADase) inhibitor as an active ingredient. The NAD glycohydrolase (NADase) inhibitor according to the present invention has the effect of reducing blood glucose levels and promoting insulin secretion and thus can be effectively used as a therapeutic agent for diabetes, particularly type II diabetes.

## Description

### CROSS-REFERENCE TO RELATED APPLICATION

This application claims priority to and the benefit of Korean Patent Application No. 10-2011-108216, filed on October, 21, 2011, the disclosure of which is incorporated herein by reference in its entirety.

### BACKGROUND

### 1. Field of the Invention

The present invention relates to a composition for preventing or treating diabetes, comprising an NAD glycohydrolase (NADase) inhibitor as an active ingredient.

### 2. Discussion of Related Art

NAD glycohydrolases (NADases) catalyze the hydrolysis of NAD to ADP-ribose (ADPR) and nicotinamide and are members of a large enzyme superfamily, which include ADP-ribosyltransferases (ARTs), poly-ADP-ribose polymerases (PARPs), ADP-ribosyl cyclases (ADPR-cyclases), and Sirtuins (Sirts). These enzymes catalyze specific reactions using NAD as a substrate.

NADase activity in mammals was identified and characterized from rabbit erythrocytes (Kim et al., Arch. Biochem.Biophys.305:147-152, 1993). NADase was anchored to the plasma membrane via a glycosylphosphatidylinositol (GPI) linkage and could be solubilized by incubation with *Bacillus cereus* phosphatidylinositol-specific phospholipase C (PI-PLC) (Kim et al., Biochem.Biophys.Acta.965:76-81, 1988).

Upon the hydrolysis of NAD, NADases catalyze a reaction for producing nicotinamide and ADP-ribose. ADP-ribosylation regulates cellular functions including cell signaling and apoptosis.

However, nucleotide sequences of mammalian NADases have not yet been determined, and functions and physiological roles of NADases in mammalian cells have not been identified. Accordingly, the present inventors have obtained a partial amino acid sequence of mammalian NADase from rabbit erythrocytes by purification for the first time. Moreover, the present inventors have obtained a full-length nucleotide sequence by performing PCR using the amino acid sequence and deposited the nucleotide sequence in the genetic sequence database of the National Institutes of Health (GenBank accession No. JN798515).

Meanwhile, diabetes is a disease in which glucose in blood is excreted through urine and is a chronic degenerative disease which is not completely cured. Recently, due to the change in dietary habit and lack of exercise, there are significant changes in energy metabolism of the human body, and thus the occurrence of chronic degenerative diseases such as diabetes, etc. tends to increase. It has been reported that the prevalence of diabetes is 5 to 10% in Korea and continuously increases. In the United States, the prevalence of diabetes has increased 6 times over the past 40 years, and it is predicted that the number of diabetic patients will reach 26 million by 2050.

It has been known that the cause of type II (non-insulin dependent) diabetes, which accounts for over 95% of diabetes cases, is associated with both insulin secretion deficiency and insulin resistance. Insulin secretion deficiency is a condition in which an appropriate amount of insulin is not secreted from pancreatic beta cells in response to the concentration of blood glucose, which include both a decrease in the amount of beta cells that secrete insulin and a functional deficiency of insulin. Insulin resistance is a condition in which the secreted insulin reaches a target organ through the bloodstream, but the insulin activity and sensitivity are reduced in its target cells. In general, the reason for this is due to a signal transduction disorder after binding to a plasma membrane receptor, and the cause of insulin resistance includes genetic factors, obesity, physical hypoactivity, hyperglycemia, dyslipidemia, etc. When there is insulin resistance, a greater amount of insulin should be secreted to overcome the resistance and, on the contrary, the hyperglycemia resulting from insufficient insulin may worsen the insulin resistance again.

At present, the non-insulin dependent diabetes is treated with diet changes, exercise, sulfonylurea agents, biguanide drugs, α-glucosidase inhibitors, insulin, etc., and meglitinide or thiazolidinone drugs, insulin with various delivery systems, etc. have been developed and applied to clinical practice through extensive research on the development of new drugs. However, these drugs have low efficacy or cause many side effects such as dyshepatia, hypoglycemia, lacticacidemia, etc. Thus, the development of a therapeutic agent for diabetes, which reduces the occurrence of these side effects, improves metabolic disorders, and has excellent safety, is urgently needed.

Thus, the present inventors have conducted extensive research to solve the above-described problems associated with the prior art and develop a more effective therapeutic agent for diabetes, and found that NADase knockdown resulted in a decrease in blood glucose and an increase in insulin secretion from diabetic animal models, thus completing the present invention.

### SUMMARY OF THE INVENTION

Accordingly, an object of the present invention is to provide a pharmaceutical composition for preventing or treating diabetes, comprising an expression inhibitor or activity inhibitor of NAD glycohydrolase (NADase) as an active ingredient.

Another object of the present invention is to provide a composition for diagnosing diabetes, comprising an agent for measuring an mRNA or protein level of NAD glycohydrolase (NADase).

Still another object of the present invention is to provide a kit for diagnosing diabetes, comprising an agent for measuring an mRNA or protein level of NAD glycohydrolase (NADase).

Yet another object of the present invention is to provide a method for screening a candidate agent for preventing or treating diabetes, comprising measuring the level of expression or activity of NAD glycohydrolase (NADase).

In order to achieve the above objects, the present invention provides a pharmaceutical composition for preventing or treating diabetes, comprising an expression inhibitor or activity inhibitor of NAD glycohydrolase (NADase) as an active ingredient.

Moreover, the present invention provides a composition for diagnosing diabetes, comprising an agent for measuring an mRNA or protein level of NAD glycohydrolase (NADase).

Furthermore, the present invention provides a kit for diagnosing diabetes, comprising an agent for measuring an mRNA or protein level of NAD glycohydrolase (NADase).

In addition, the present invention provides a method for screening a candidate agent for preventing or treating diabetes, comprising measuring the level of expression or activity of NAD glycohydrolase (NADase).

### BRIEF DESCRIPTION OF THE DRAWINGS

The above and other objects, features and advantages of the present invention will become more apparent to those of ordinary skill in the art by describing in detail exemplary embodiments thereof with reference to the accompanying drawings, in which:
FIG. 1 is a diagram showing the amino acid and nucleotide sequences of NAD glycohydrolase (NADase) obtained from a cDNA library of rabbit reticulocytes;
FIG. 2 is a diagram showing the amino acid and nucleotide sequences of NAD glycohydrolase (NADase) obtained from a cDNA library of mouse bone marrow cells;
FIG. 3 is a diagram showing the results of RT-PCR analysis of NADase in rabbit reticulocytes, mouse bone marrow cells, mouse pancreatic cells, and mouse pancreatic β-cells (MIN6);
FIG. 4 is a diagram showing NAD levels upon NADase overexpression or knockdown in MIN6 cell line;
FIG. 5 is a diagram showing insulin secretion rates in response to the concentration of glucose upon NADase overexpression or knockdown in MIN6 cell line; and
FIG. 6 is a diagram showing the therapeutic effects of diabetes when a vector expressing shRNA capable of specifically inhibiting the synthesis of NADase is administered to diabetic mouse models.

### DETAILED DESCRIPTION OF EXEMPLARY EMBODIMENTS

Hereinafter, the present invention will be described in detail.

The present invention provides a pharmaceutical composition for preventing or treating diabetes, comprising an expression inhibitor or activity inhibitor of NAD glycohydrolase (NADase) as an active ingredient.

The NAD glycohydrolase (NADase) inhibitor according to the present invention has the effect of reducing blood glucose levels and promoting insulin secretion and thus can be effectively used as a therapeutic agent for diabetes, particularly type II diabetes.

Protein of the NAD glycohydrolase (NADase) of the present invention may be derived from a mammal and may preferably be derived from a mouse (GenBank Accession No. JN798516; SEQ ID No. 1) or a rabbit (GenBank, Accession No. JN798515; SEQ ID No. 2), but not limited thereto.

The protein of the NAD glycohydrolase (NADase) of the present invention includes a protein encoded by nucleotide sequence represented by SEQ ID NO. 1 or 2 and a functional equivalent thereof. As a result of addition, substitution or deletion of an amino acid, the "functional equivalent" has protein encoded by nucleotide sequence represented by SEQ ID NO. 1 or 2 and a sequence homology of at least 70%, preferably more than 80%, more preferably more than 90%, most preferably more than 95% and includes a protein encoded by nucleotide sequence of SEQ ID NO. 1 or 2 and a protein with substantially the same biological activity.

The protein of the NAD glycohydrolase (NADase) of the present invention includes proteins with naturally occurring amino acid sequences and variants with altered amino acid sequences as well. The variants of the protein of the NAD glycohydrolase (NADase) of the present invention refers to proteins with different amino acid sequences due to deletion, insertion, non-conservative or conservative substitution, or combinations thereof of the naturally occurring amino acid sequence of the NAD glycohydrolase and at least one amino acid residue. The exchange of amino acids in proteins and peptides that do not substantially alter the activity of molecules is well known in the art (H. Neurath, R. L. Hill, The Proteins, Academic Press, New York, 1979). The most commonly occurring exchange is the exchange between amino acid residues such as Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly. If desired, the protein of the present invention may be modified by phosphorylation, sulfation, acetylation, glycosylation, methylation, famesylation, etc.

The NAD glycohydrolase protein or a variant thereof may be naturally isolated or synthesized (Merrifield, J. Amer. chem. Soc.85:2149-2156, 1963) or may be prepared by genetic recombination based on DNA sequences (Sambrook et al, Molecular Cloning, Cold Spring Harbour Laboratory Press, New York, USA, 2nd Ed., 1989).

The expression inhibitor of the NAD glycohydrolase (NADase) protein may be selected from the group consisting of an antisense nucleotide, a short hairpin RNA (shRNA), a small interfering RNA (siRNA), and a ribozyme, complementarily binding to mRNA of an NAD glycohydrolase (NADase) gene, and the activity inhibitor of the NAD glycohydrolase (NADase) protein may be selected from the group consisting of a compound, a peptide, a peptide mimetic, a substrate analogue, an aptamer, and an antibody, complementarily binding to an NAD glycohydrolase (NADase) protein, but not limited thereto.

The siRNA comprises a sense sequence of 15 to 30-mers selected from the mRNA sequence of a gene that encodes the NAD glycohydrolase (NADase) protein and an antisense sequence complementarily binding to the sense sequence. Here, the sense sequence may be composed of 25 nucleotides, but not particularly limited thereto. As described by Watson-Crick base-pair model, the nucleotide sequence is hybridized with a complementary sequence of DNA, immature-mRNA or mature-mRNA to interrupt the transmission of genetic information. A target sequence specific antisense nucleotide is exceptionally multi-functional. The antisense nucleotide is a long chain of monomers, which favors hybridization to a target RNA sequence. Numbers of reports have recently been made to prove the utility of an antisense nucleotide as a biochemical tool in study of a target protein (Rothenberg et al., J. Natl. Cancer Inst., 81:1539-1544, 1999). Great progress has been made in the fields of oligonucleotide synthesis, improved cell adhesion of oligonucleotide, target binding affinity and synthesis of nucleotide having resistance against nuclease, suggesting that an antisense nucleotide might be a new sort of a therapeutic agent.

The peptide mimetics inhibit the binding domain of the NAD glycohydrolase (NADase) protein, thus inhibiting the activity of the NAD glycohydrolase (NADase) protein. The peptide mimetics may be peptides or non-peptides and may comprise amino acids linked by non-peptide bonds such as psi bonds (Benkirane, N., et al. J. Biol. Chem., 271:33218-33224, 1996). Moreover, the peptide mimetics may be "conformationally constrained" peptides, cyclic mimetics, or cyclic mimetics comprising at least one exocyclic domain, a linking moiety (linking amino acid) and an active region. The peptide mimetics are constructed to resemble secondary structural features of the NAD glycohydrolase (NADase) protein and can mimic inhibitory features of large molecules such as antibody (Park, B. W. et al. Nat Biotechnol 18, 194-198, 2000)) or soluble receptors (Takasaki, W. et al. Nat Biotechnol 15, 1266-1270, 1997). These peptides represent novel small molecular tools that can act with potency comparable to or equivalent to the natural antagonist (Wrighton, N. C. et al. Nat Biotechnol 15, 1261-1265, 1997).

The aptamer is a single-stranded DNA or RNA molecule and can be obtained by isolating oligomers that bind to specific chemical molecules or biological molecules with high affinity and specificity by an evolutionary method using an oligonucleotide library called systematic evolution of ligands by exponential enrichment (SELEX) (C. Tuerand L. Gold, Science 249, 505 - 510, 2005; A. D. Ellington and J. W. Szostak, Nature 346, 818 - 822, 1990; M. Famulok, et. al., Acc. Chem. Res. 33, 591 - 599, 2000; D. S. Wilson and Szostak, Annu. Rev. Biochem. 68, 611-647, 1999). The aptamer can specifically bind to a target to regulate its activity and can inhibit the function of the target through bonds, for example.

The antibody specifically and directly binds to the NAD glycohydrolase (NADase) to effectively inhibit its activity. The antibody that specifically binds to the NAD glycohydrolase (NADase) may be a polyclonal antibody or monoclonal antibody. The antibody that specifically binds to the NAD glycohydrolase (NADase) may be prepared by a method known to those skilled in the art, and a commercially available NAD glycohydrolase (NADase) antibody may be used. The antibody may be prepared by injecting the NAD glycohydrolase (NADase) protein as an immunogen into a host according to a conventional method known to those skilled in the art. The host may include mammals such as mice, rats, sheep, rabbits, etc. The immunogen may be injected intramuscularly, intraperitoneally, or subcutaneously or may be injected with an adjuvant to enhance antigenicity. Blood samples may be taken from the host at regular intervals and serum exhibiting titer and specificity to the antigen may be collected to separate an antibody therefrom.

The composition of the present invention may be used in conjunction with a therapeutic agent for diabetes, as is conventionally known in the art, as well as the expression inhibitor or activity inhibitor of NAD glycohydrolase (NADase) as an active ingredient.

The composition of the present invention may be formulated into an appropriate form in conjunction with a pharmaceutically acceptable carrier. The "pharmaceutically acceptable carrier" refers to a carrier that is biologically acceptable and does not cause allergic reactions such as gastroenteric trouble and dizziness or other adverse reactions, when administered to human. Examples of the pharmaceutically acceptable carrier may include carriers for oral administration such as lactose, starch, cellulose derivatives, magnesium stearate, and stearic acid, and carriers for parenteral administration such as water, suitable oil, saline solution, aqueous glucose, and glycol. The pharmaceutically acceptable carrier may further comprise a stabilizer and a preservative. The suitable stabilizer may comprise antioxidants such as sodium bisulfite, sodium sulfite, and ascorbic acid. The suitable preservative may comprise benzalkonium chloride, methyl- or propyl-paraben, and chlorobutanol. Other pharmaceutically acceptable carriers can be found in (Remington's Pharmaceutical Sciences, 19th ed., Mack Publishing Company, Easton, PA, 1995).

For oral administration, the pharmaceutical composition of the invention may be formulated into powder, granule, tablet, pill, sugar-coated tablet, capsule, liquid, gel, syrup, suspension, wafer, etc. together with a suitable carrier for oral administration according to a method known to those skilled in the art. Examples of suitable carriers may include: saccharides such as lactose, dextrose, sucrose, sorbitol, mannitol, xylitol, erythritol, maltitol, etc.; starches such as corn starch, wheat starch, rice starch, potato starch, etc.; celluloses such as cellulose, methyl cellulose, sodium carboxymethyl cellulose, hydroxypropylmethyl cellulose, etc.; and fillers such as gelatin, polyvinyl pyrrolidone, etc. If necessary, a disintegrant such as crosslinked polyvinyl pyrrolidone, agar, alginic acid, sodium alginate, etc. may be added thereto. In addition, the pharmaceutical composition may further comprise anticoagulants, lubricants, wetting agents, flavouring agents, emulsifiers, preservatives, etc. For parenteral administration, the pharmaceutical composition of the present invention may be formulated, for example, into injection, transdermal system, or nasal inhaler together with a suitable parenteral carrier according to a method known in the art. The injection should be sterilized and be protected from contamination of microorganisms such as bacteria and fungi. In the case of the injection, examples of suitable carriers may include, but not limited to, a solvent or dispersion medium containing, for example, water, ethanol, polyol (such as glycerol, propylene glycol, and liquid polyethylene glycol), mixtures thereof, and/or vegetable oil. More preferably, the suitable carriers may include, Hanks's solution, Ringer's solution, phosphate buffered saline (PBS) containing triethanolamine, sterile water for injection, or isotonic solution such as 10% ethanol, 40% propylene glycol, and 5% dextrose. The injection may further include various antibacterial and antifungal agents such as paraben, chlorobutanol, phenol, sorbic acid, thimerosal, etc. for protection from microbial contamination. Further, the injection may include an isotonic agent such as sugar or sodium chloride in most cases. The transdermal administration system may be in the form of ointment, cream, lotion, gel, topical solution, paste, liniment, aerosol, etc. As used herein, "transdermal administration" refers to the delivery of an effective amount of active ingredient included in the pharmaceutical composition topically into the skin. These formulations are described in the Remington's Pharmaceutical Science, 15th Edition, 1975, Mack Publishing Company, Easton, Pa., which is well known in the pharmaceutical chemistry field. The inhaler may be in the form of a pressurized pack or an aerosol spray delivered from a nebulizer using an adequate propellant compound, e.g. dichlorofluoromethane, trichlorofluoromethane, dichlorotetrafluoroethane, carbon dioxide, etc. for easier delivery. The pressurized aerosol may be equipped with a valve for delivering a unit dosage. A gelatin capsule or cartridge used in the inhaler may include a powder mixture of lactose, starch or other matrix. Other pharmaceutically acceptable carriers may be consulted from Remington's Pharmaceutical Sciences, 19th ed., Mack Publishing Company, Easton, Pa., 1995.

Nucleotide or nucleic acid used in the present invention may be formulated for oral, local, parenteral, intranasal, intravenous, intramuscular, subcutaneous, ophthalmic, or transdermal administration. It is more preferred to prepare nucleic acid or vector as an injectable formulation. For direct injection, the injectable composition may be mixed with a pharmaceutically acceptable medium. The composition of the present invention may also include a freeze-dried composition which enables the preparation of an injectable solution with the addition of sterilized isotonic solution, distilled water, or appropriate physiological saline. As used herein, the term "pharmaceutically effective amount" refers to an amount that causes more reactions, compared to a control group and, preferably, refers to an amount sufficient for preventing or treating diabetes. The pharmaceutically effective amount of the composition of the present invention is about 0.0001 to 100 mg/kg per day, and preferably about 0.001 to 10 mg/kg per day. Preferably, the composition of the present invention may be administered once or several times a day. The dosage of nucleic acid may be regulated according to diverse parameters, particularly a gene or a vector, administration method, target disease and required treatment period, etc. In addition, the dosage of nucleic acid may vary according to a patient's weight, age, gender, health condition, diet, administration time, administration method, excretion, and severity of a disease.

Also, the present invention provides a composition for diagnosing diabetes, comprising an agent for measuring an mRNA or protein level of NAD glycohydrolase (NADase).

As used herein, the term "diagnosis" refers to the process of determining the presence or absence of a pathological condition. With respect to the objects of the present invention, the diagnosis is to determine the incidence of diabetes.

Moreover, the present invention provides a kit for diagnosing diabetes, comprising an agent for measuring an mRNA or protein level of NAD glycohydrolase (NADase). Preferably, the kit for diagnosing diabetes may further comprise one or more compositions, solutions, or devices which are suitable for the analysis.

Furthermore, the present invention provides a method for screening a candidate agent for preventing or treating diabetes, comprising measuring the level of expression or activity of NAD glycohydrolase (NADase).

More specifically, the present invention provides a method for screening a candidate agent for preventing or treating diabetes, the method comprising the steps of: (a) treating an NAD glycohydrolase (NADase) protein expression cell line with a subject material; (b) measuring the level of expression or activity of NAD glycohydrolase (NADase) in the cell line; and (c) selecting a subject material with a decreased level of expression or activity of the NAD glycohydrolase (NADase) compared to a control group which has not been treated with the subject material.

In the above method, the level of expression of the protein in step (b) may preferably be measured by a method selected from the group consisting of immunoprecipitation, radioimmunoassay (RIA), enzyme-linked immunosorbent assay (ELISA), immunohistochemistry, reverse transcriptase PCR (RT-PCR), Western-blotting, and flow cytometry (FACS), but all methods of measuring the amount of transcriptome known to those skilled in the art or the amount of protein coated with the transcriptome may be used.

Moreover, in the above method, the level of activity of the protein in step (b) may preferably be measured by a method selected from the group consisting of SDS-PAGE, immunofluorescence, ELISA, mass spectrometry, and protein chip.

Hereinafter, preferred Examples will be provided to facilitate understanding of the present invention. However, the following Examples are provided for better understanding of the present invention only, and the scope of the present invention is not limited by the following Examples.

### Example 1: Analysis of NADase nucleotide sequence

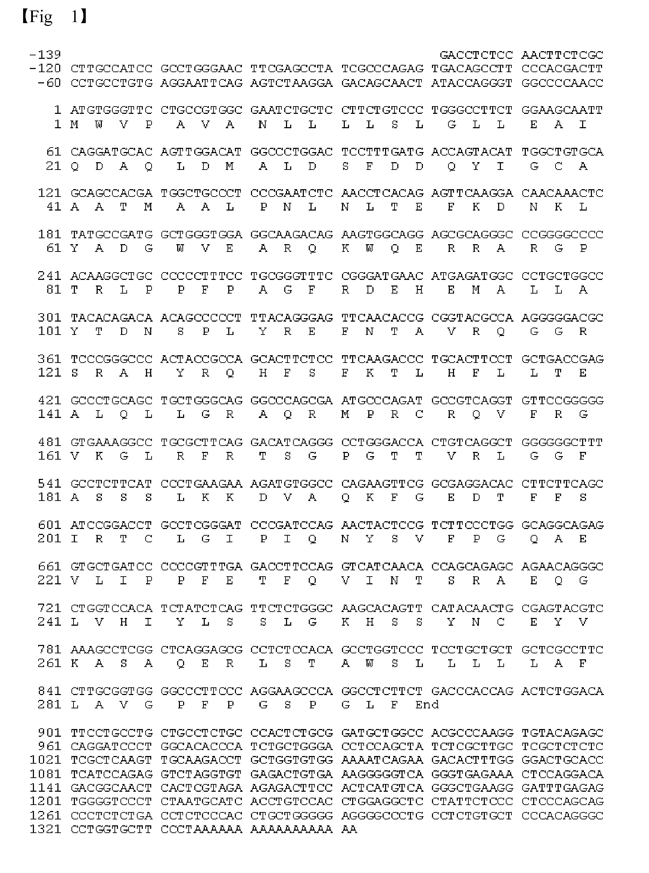

Total RNA was isolated from rabbit reticulocytes and primers of rabbit reticulocyte NADase were designed on the basis of the sequence shown in the following table 1, thus performing RT-PCR. Forty cycles of amplification were carried out as follows: 1 min at 94 °C; 1 min at 62 °C; and 1 min at 72 °C. The PCR product was ligated into a T&A vector to obtain plasmids, which were verified by nucleotide sequence analysis (Genotech, Daejeon, Korea). By the same method, total RNA was isolated from mouse bone-marrow cells, mouse pancreatic islet cells, and mouse pancreatic β-cells (MIN6), and the nucleotide sequences of NADase was determined. The results are shown in FIGS. 1 to 3.

**[Table 1]**

| | |
|---|---|
| NA20-F | 5'-CGCTCGAGGAATTCCATTCAGGATGCACAGTTGGACATG-3' |
| NA243-R | 5'-CCCAAGCTTGTCGACGTGGACCAGGCCCTGTTCTGC-3' |
| NA1-F | 5'-CGGAATTCCATGTGGGTTCCTGCCGTGGCG-3' |
| NA293-R | 5'-ACGCGTCGACGAAGAGGCCTGGGCTTCCTGGGA-3' |

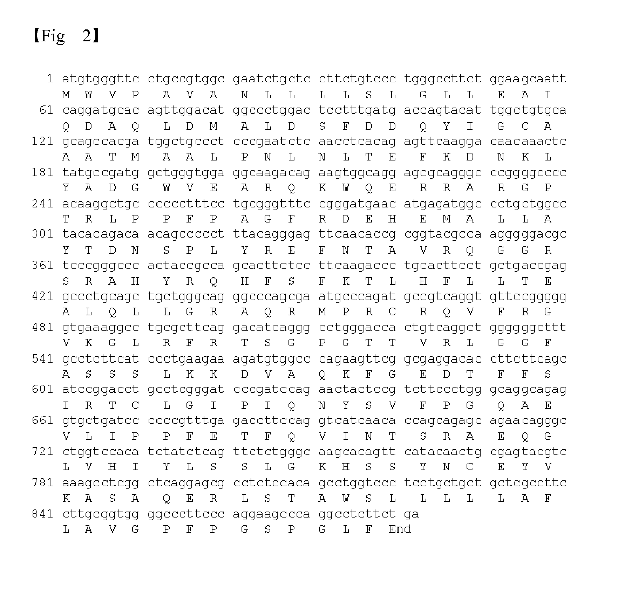

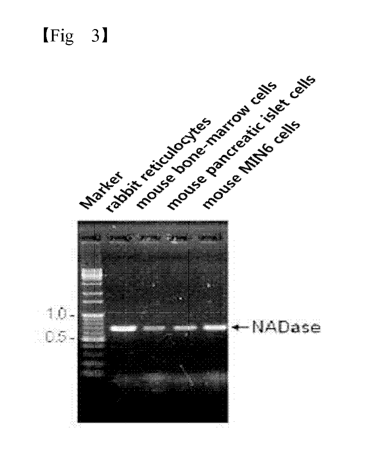

As shown in FIGs. 1 and 2, the NADase sequences obtained from the rabbit reticulocytes and mouse bone-marrow cells were determined. Moreover, as shown in FIG. 3, it was found that NADase genes were present in the rabbit reticulocytes, mouse bone marrow cells, mouse pancreatic islet cells, and MIN6 cells.

### Example 2: Change in NAD concentration according to regulation of expression of NADase in MIN6 cells

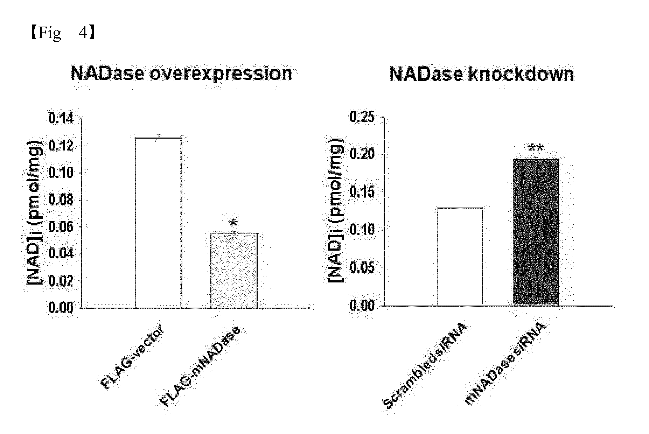

In order to determine the change in NAD concentration according to the regulation of the expression of NADase in MIN6 cells, the following test was carried out by a method reported by Graeff R. et al. (Biochem. J. 367:163-168, 2002). First, mouse NADase was ligated into a FLAG-CMV-2 vector (Sigma-Aldrich, MO, USA) to prepare FLAG-NADase plasmids. MIN6 cells were transfected with the plasmids using Lipofectamine (Invitrogen, CA, USA) to induce NADase overexpression. Moreover, siRNA oligomers (Genolution, Seoul, Korea) against mouse NADase were prepared and MIN6 cells were transfected with the siRNA oligomers using Lipofectamine to induce NADase knockdown. The expression- or knockdown-induced MIN6 cells were treated with 0.6 M trichloroacetic acid. Proteins were removed by centrifugation at 15,000 Xg. Then, the supernatants (0.1 ml) containing NAD were incubated with the same amount of ethanol (2%), alcohol dehydrogenase (100 µg/ml), resazurin (20 µM), diaphorase (10 µg/ml) FMN (10 µM), nicotinamide (10 mM), bovine serum albumin (BSA, 0.1 mg/ml), and sodium phosphate buffer (100 mM, pH 8.0) for 2 to 4 hours. The fluorescence of reactants was measured at an excitation wavelength of 544 nm and at an emission wavelength of 590 nm using a spectrophotofluorometer (Hitachi). The results are shown in FIG. 4.

As shown in FIG. 4, it was found that the NAD concentration in the NADase-overexpressing MIN6 cells was significantly reduced (P < 0.001), and the NAD concentration in the NADase-knockdown MIN6 cells significantly increased (P < 0.0005).

### Example 3: Change in insulin secretion according to regulation of expression of NADase in MIN6 cells

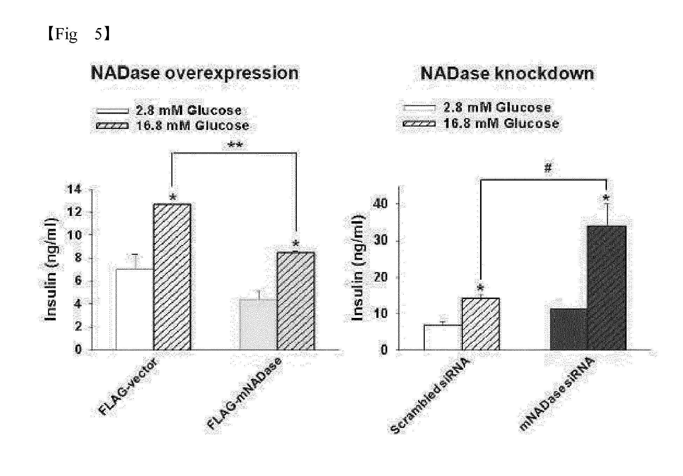

In order to determine the change in insulin secretion according to the regulation of the expression of NADase in MIN6 cells, the following test was carried out using a rat insulin RIA kit (Millipore, CA, USA). First, the mouse NADase-overexpressing or knockdown MIN6 cells were washed with modified Krebs-Ringer (KR) buffer (119 mM NaCl, 4.74 mM KCl, 2.54 mM CaCl₂, 1.19 mM MgCl₂, 1.19 mM KH₂PO₄ 25 mM NaHCO₃ and 10 mM Hepes, pH 7.4). The MIN6 cells were treated with KR buffer containing 0.1% BSA and 2.8 mM glucose and stabilized at 37°C for 30 min. Subsequently, the overexpressing or knockdown MIN6 cells were incubated 37°C for 4 hours in KR buffer containing 2.8 mM glucose and 0.1% BSA or in KR buffer containing 16.8 mM glucose and 0.1 % BSA, and then the amount of insulin secreted into the KR buffer was measured. The results are shown in FIG. 5.

As shown in FIG. 5, it was found that the insulin secretion in the MIN6 cells treated with 16.8 mM glucose was increased about 2 times (P < 0.05), compared to the MIN6 cells treated with 2.8 mM glucose. On the contrary, there was no difference in the insulin secretion between the NADase-overexpressing MIN6 cells treated with 16.8 mM glucose and the NADase-non-overexpressing MIN6 cells treated with 2.8 mM glucose. Moreover, as a result of NADase knockdown using siRNA, it was found that the insulin secretion significantly increases (P < 0.05) when the control group and the NADase knockdown group treated with siRNA were treated with 2.8 mM glucose and 16.8 mM glucose, respectively.

### Example 4: Therapeutic effect of diabetes according to regulation of expression of NADase in diabetic mouse models

In order to determine the therapeutic effect of diabetes according to the regulation of the expression ofNADase in diabetic mouse models, the following test was carried out. First, NADase in pancreatic cells was knocked down using a lentiviral shRNA vector (Carlson, et al., Nature, 454:528-532, 2008; Leung et al., Endocrinology, 146:4766-4775, 2005). More specifically, lentiviral shRNA plasmids were obtained by ligating a CMV promoter, an EGFP, and a mouse insulin promoter cassette to a downstream of a mouse U6 promoter in a lentiviral shRNA vector (pLKO.1-puro, Sigma-aldrich, MO, USA) and ligating NADase short hairpin RNA (shRNA) to an upstream of the mouse U6 promoter. Then, 293 FT cells (Invitrogen, CA, U.S.A.) were transfected with the lentiviral shRNA plasmids and a lentiviral packaging mix (MISSION Lentiviral packaging Mix, Sigma-aldrich, MO, USA) and incubated for a predetermined time, thus isolating lentiviral particles from the supernatant. The isolated lentiviral particles were concentrated using the Lenti-X concentrator (ClonTech, CA, USA)) and resuspended to each well at a concentration of 1 x 10⁹ transducing units (T.U.)/100 µl.

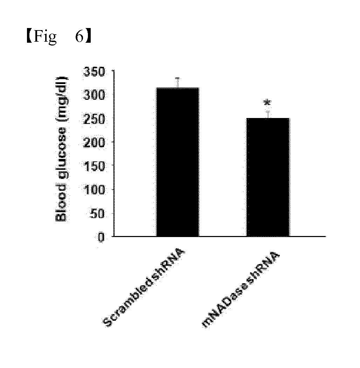

Thereafter, the lentiviral suspension obtained by the above method was injected into a tail vein of each 7-week old type II diabetic mouse (C57BL/KsJ-db/db Jcl, Charls River, Tokyo, Japan) to determine the therapeutic effect of diabetes. The lentiviral suspension was administered once a week for a total of 3 weeks, and the weight and blood glucose were measured at 4 weeks. Scrambled shRNA was administered to a control group. The results are shown in FIG. 6.

As shown in FIG. 6, it was found that there was no significant difference between the experimental groups (no data is shown), but the experimental group to which the lentivirus expressing shRNA capable of inhibiting NADase was applied showed a significant blood glucose lowering effect (P < 0.05) compared to the control group, from which it can be concluded that the NADase inhibitor has the therapeutic effect of diabetes.

Next, Preparation Examples of the pharmaceutical composition comprising the composition of the present invention will be described, but these Preparation Examples are for illustrative purpose only, and the present invention is not limited by these Examples.

### Preparation Example 1: Preparation of Powders

| NAD glycohydrolase (NADase) inhibitor: 20 mg | |
|---|---|
| Lactose: | 100 mg |
| Talc: | 10 mg |

The above ingredients are mixed and filled in airtight bags to prepare powders.

### Preparation Example 2: Preparation of Tablets

| NAD glycohydrolase (NADase) inhibitor: 10 mg | |
|---|---|
| Corn starch: | 100 mg |
| Lactose: | 100 mg |
| Magnesium stearate: | 2 mg |

The above ingredients are mixed and compressed into tablets by a typical preparation method of tablets.

### Preparation Example 3: Preparation of Capsules

| NAD glycohydrolase (NADase) inhibitor: 10 mg | |
|---|---|
| Crystalline cellulose: | 3 mg |
| Lactose: | 14.8 mg |
| Magnesium stearate: | 2 mg |

The above ingredients are mixed and filled in gelatin capsules to prepare capsules by a typical preparation method of capsules.

### Preparation Example 4: Preparation of Injections

| NAD glycohydrolase (NADase) inhibitor: 10 mg | |
|---|---|
| Mannitol: | 180 mg |
| Sterile distilled water for injection: | 2,974 mg |
| Na₂HPO_{4·}2H₂O: | 26 mg |

Injections are prepared with the above ingredients per ampoule (2 ml) by a typical preparation method of injections.

### Preparation Example 5: Preparation of liquid medicines

NAD glycohydrolase (NADase) inhibitor: 20 mg
Isomerized sugar 10 g
Mannitol: 5 mg
Purified water: Suitable amount

Liquid medicines are prepared with the above ingredients by a typical preparation method of liquid medicines.

As described above, the NAD glycohydrolase (NADase) inhibitor according to the present invention has the effect of reducing blood glucose levels and promoting insulin secretion and thus can be effectively used as a therapeutic agent for diabetes, particularly type II diabetes.

It will be apparent to those skilled in the art that various modifications can be made to the above-described exemplary embodiments of the present invention without departing from the spirit or scope of the invention. Thus, it is intended that the present invention covers all such modifications provided they come within the scope of the appended claims and their equivalents.

## Claims

1. A pharmaceutical composition for preventing or treating diabetes, comprising an expression inhibitor or activity inhibitor of NAD glycohydrolase (NADase) as an active ingredient.

2. The pharmaceutical composition of claim 1, wherein the NAD glycohydrolase (NADase) is derived from a mammal.

3. The pharmaceutical composition of claim 2, wherein the NAD glycohydrolase (NADase) is encoded by a nucleotide sequence of SEQ. ID No. 1 or 2.

4. The pharmaceutical composition of claim 1, wherein the diabetes is type II diabetes.

5. The pharmaceutical composition of claim 1, wherein the expression inhibitor is selected from the group consisting of an antisense nucleotide, a small interfering RNA (siRNA), and a short hairpin RNA (shRNA), complementarily binding to mRNA of an NAD glycohydrolase (NADase) gene.

6. The pharmaceutical composition of claim 1, wherein the activity inhibitor is selected from the group consisting of a compound, a peptide, a peptide mimetic, an aptamer, and an antibody, complementarily binding to an NAD glycohydrolase (NADase) protein.

7. A composition for diagnosing diabetes, comprising an agent for measuring an mRNA or protein level of NAD glycohydrolase (NADase).

8. A kit for diagnosing diabetes, comprising an agent for measuring an mRNA or protein level of NAD glycohydrolase (NADase).

9. A method for screening a candidate agent for preventing or treating diabetes, the method comprising the steps of:
(a) treating an NAD glycohydrolase (NADase) protein expression cell line with a subject material;
(b) measuring the level of expression or activity of NAD glycohydrolase (NADase) in the cell line; and
(c) selecting a subject material with a decreased level of expression or activity of the NAD glycohydrolase (NADase) compared to a control group which has not been treated with the subject material.

10. The method of claim 9, wherein in step (b), the expression of NADase is measured by at least one method selected from the group consisting of immunoprecipitation, radioimmunoassay (RIA), enzyme-linked immunosorbent assay (ELISA), immunohistochemistry, reverse transcriptase PCR (RT-PCR), Western-blotting, and flow cytometry (FACS).

11. The method of claim 9, wherein in step (b), the level of activity of NADase is measured by at least one method selected from the group consisting of SDS-PAGE, immunofluorescence, ELISA, mass spectrometry, and protein chip.


<110>    INDUSTRIAL COOPERATION FOUNDATION CHONBUK NATIONAL UNIVERSITY

<120> Composition for preventing or treating diabetes comprising NAD
glycohydrolase inhibitor as active ingredient

<130>    J60-B-39400 EP

<150>    KR11/108216
<151>    2011-10-21

<160>    2

<170>    KopatentIn 2.0

<210>    1
<211>    882
<212>    DNA
<213>    NAD glycohydrolase from mouse
<400>    1
atgtgggttc ctgccgtggc gaatctgctc cttctgtccc tgggccttct ggaagcaatt         60

caggatgcac agttggacat ggccctggac tcctttgatg accagtacat tggctgtgca        120

gcagccacga tggctgccct cccgaatctc aacctcacag agttcaagga caacaaactc        180

tatgccgatg gctgggtgga ggcaagacag aagtggcagg agcgcagggc ccggggcccc        240

acaaggctgc ccccctttcc tgcgggtttc cgggatgaac atgagatggc cctgctggcc        300

tacacagaca acagccccct ttacagggag ttcaacaccg cggtacgcca agggggacgc        360

tcccgggccc actaccgcca gcacttctcc ttcaagaccc tgcacttcct gctgaccgag        420

gccctgcagc tgctgggcag ggcccagcga atgcccagat gccgtcaggt gttccggggg        480

gtgaaaggcc tgcgcttcag gacatcaggg cctgggacca ctgtcaggct ggggggcttt        540

gcctcttcat ccctgaagaa agatgtggcc cagaagttcg gcgaggacac cttcttcagc        600

atccggacct gcctcgggat cccgatccag aactactccg tcttccctgg gcaggcagag        660

gtgctgatcc ccccgtttga gaccttccag gtcatcaaca ccagcagagc agaacagggc        720

ctggtccaca tctatctcag ttctctgggc aagcacagtt catacaactg cgagtacgtc        780

aaagcctcgg ctcaggagcg cctctccaca gcctggtccc tcctgctgct gctcgccttc        840

cttgcggtgg ggcccttccc aggaagccca ggcctcttct ga                           882


<210>    2
<211>    1513
<212>    DNA
<213>    NAD glycohydrolase from rabbit


<400>    2
   gacctctcca acttctcgcc ttgccatccg cctgggaact tcgagcctac tgcccagagt         60
   gacagccttc ccacgacttc ctgcctgtga ggaattcaga gtctaaggag acagcaacta        120
   taccagggtg gccccaacca tgtgggttcc tgccgtggcg aatctgctcc ttctgtccct        180
   gggccttctg gaagcaattc aggatgcaca gttggacatg gccctggact cctttgatga        240
   ccagtacatt ggctgtgcag cagccacgat ggctgccctc ccgaatctca acctcacaga 300
   gttcaaggac aacaaactct atgccgatgg ctgggtggag gcaagacaga agtggcagga 360
   gcgcagggcc cggggcccca caaggctgcc cccctttcct gcgggtttcc gggatgaaca 420
   tgagatggcc ctgctggcct acacagacaa cagccccctt tacagggagt tcaacaccgc 480
   ggtacgccaa gggggacgct cccgggccca ctaccgccag cacttctcct tcaagaccct 540
   gcacttcctg ctgaccgagg ccctgcagct gctgggcagg gcccagcgaa tgcccagatg 600
   ccgtcaggtg ttccgggggg tgaaaggcct gcgcttcagg acatcagggc ctgggaccac 660
   tgtcaggctg gggggctttg cctcttcatc cctgaagaaa gatgtggccc agaagttcgg 720
   cgaggacacc ttcttcagca tccggacctg cctcgggatc ccgatccaga actactccgt 780
   cttccctggg caggcagagg tgctgatccc cccgtttgag accttccagg tcatcaacac 840
   cagcagagca gaacagggcc tggtccacat ctatctcagt tctctgggca agcacagttc 900
   atacaactgc gagtacgtca aagcctcggc tcaggagcgc ctctccacag cctggtccct 960
   cctgctgctg ctcgccttcc ttgcggtggg gcccttccca ggaagcccag gcctcttctg 1020
   acccaccaga ctctggacat tcctgcctgc tgcctctgcc cactctgcgg atgctggcca 1080
   cgcccaaggt gtacagagcc aggatccctg gcacacccat ctgctgggac ctccagctat 1140
   ctcgcttgct cgctctctct cgctcaagtt gcaagacctg ctggtgtgga aaatcagaag 1200
   acactttggg gactgcacct catccagagg tctaggtgtg agactgtgaa agggggtcag 1260
   ggtgagaaac tccaggacag acggcaactc actcgtagaa gagacttcca ctcatgtcag 1320
   ggctgaaggg atttgagagt ggggtccctc taatgcatca cctgtccacc tggaggctcc 1380
   tattctcccc tcccagcagc cctctctgac ctctcccacc tgctggggga ggggccctgc 1440
   ctctgtgctc ccacagggcc ctggtgcttc cctaaaaaaa aaaaaaaaaa aatcgtagtc 1500
   gcagcattca cag 1513